Europäisches Patentamt

European Patent Office

Office européen des brevets

(19)

(11) EP 0 603 773 B1

(12) EUROPÄISCHE PATENTSCHRIFT

(45) Veröffentlichungstag und Bekanntmachung des Hinweises auf die Patenterteilung:
18.06.1997 Patentblatt 1997/25

(51) Int. Cl.$^6$: **B01J 20/12**, A01K 1/015

(21) Anmeldenummer: 93120463.0

(22) Anmeldetag: 18.12.1993

(54) **Verfahren zur Herstellung von Sorptionsmitteln zur Aufnahme von Flüssigkeiten**

Process for the preparation of a sorbent for retaining fluids

Procédé de préparation d'un sorbant pour le recueil de fluides

(84) Benannte Vertragsstaaten:
AT DE DK ES FR GB GR IT NL SE

(30) Priorität: 21.12.1992 DE 4243389

(43) Veröffentlichungstag der Anmeldung:
29.06.1994 Patentblatt 1994/26

(73) Patentinhaber: SÜD-CHEMIE AG
D-80333 München (DE)

(72) Erfinder:
• Ahlers, Rolf Dr
D-84036 Landshut (DE)
• Eisgruber, Max
D-84079 Bruckberg (DE)
• Hähn, Reinhard Dr.
D-84186 Vilsheim (DE)
• Haubensak, Otto Dr.
D-83098 Brannenburg (DE)
• Schall, Norbert Dr.
D-85465 Langenpreising (DE)

(74) Vertreter: Reitzner, Bruno, Dr. et al
Patentanwälte Dipl.-Ing. R. Splanemann
Dr. B. Reitzner, Dipl.-Ing. K. Baronetzky
Tal 13
80331 München (DE)

(56) Entgegenhaltungen:
EP-A- 0 424 001      US-A- 3 240 616
US-A- 3 408 305      US-A- 4 641 605

**Beschreibung**

Die Erfindung betrifft ein Verfahren zur Herstellung von Sorptionsmitteln zur Aufnahme von Flüssigkeiten. Diese Sorptionsmittel sind zur Aufnahme beliebiger Flüssigkeiten geeignet; sie eignen sich aber insbesondere als Streumittel für Heimtiere, weshalb sich die nachfolgenden Ausführungen insbesondere auf dieses Anwendungsgebiet beziehen.

Die Heimtierhaltung in einer weitgehend verstädterten Umwelt ist in zunehmendem Maß mit der Verwendung von Streumaterialien verbunden. Diese haben die Aufgabe, die von den Tieren abgegebenen Flüssigkeiten aufzusaugen und dabei möglichst die Geruchsentwicklung zu hemmen oder zu verhindern. Sie sollen ferner den halbfesten, feuchten tierischen Exkrementen und Ausscheidungen Feuchtigkeit entziehen, um damit die Geruchsentwicklung zu vermindern. Gute Streumaterialien haben darüberhinaus die Eigenschaft, die abgegebenen oder entzogenen Flüssigkeiten unter Bildung eines kompakten Klumpens aufzunehmen, der in einfacher und sparsamer Weise entfernt werden kann. Eine optimale Tierstreu hat somit ein hohes Saugvermögen.

Die auf dem Markt anzutreffenden Produkte sind einmal organische Substanzen, wie Stroh, Sägemehl, Holzspäne, Rinden, poröse Kunststoffperlen, geshreddertes Papier, Cellulosefasern, Agrarabfälle, Polyacrylate usw.; diese werden allein oder in Mischungen mit anorganischen Materialien eingesetzt. Der Nachteil der organischen Streumaterialien ist oftmals die nicht zufriedenstellende Klumpenbildung bzw. ihre Konsistenz sowie ihre Neigung zur bakteriellen Zersetzung, insbesondere im Zusammenwirken mit Feuchtigkeit.

Aus der JP-A-60-258 101 sind in Wasser dispergierbare Granulate als Schädlingsbekämpfungsmittel bekannt, die als Träger ein Gemisch aus Bentonit und Diatomeenerde sowie eine kleine Menge an Alkaliposphat enthalten. Es handelt sich hierbei nur um mechanische Gemische. Eine Aktivierung des Bentonits findet nicht statt.

Aus der JP-A-58-128 146 ist ein Sorptionsmittel für flüchtige anorganische Hydride bekannt, das durch Imprägnieren von festen silikatischen Trägern, wie Diatomeenerde, Calciumsilicat oder Bentontit mit wäßrigen Lösungen von Alkalien, wie NaOH, KOH oder Ca(OH)$_2$ erhalten wird. Es findet keine Verknetung und Aktivierung statt.

Aus der JP-A-53 065 283 ist es bekannt, Verunreinigungen in Abgasen durch ein Granulat aus Bentonit, Kaliumpermanganat, Natriumhydroxid, Zement als Bindemittel und gegebenenfalls Aktivkohle zu entfernen. Das Natriumhydroxid wird nicht mit dem Bentonit verknetet und wird nicht zu dessen Aktivierung verwendet.

Aus der JP-A-52-065 765 ist ein Verfahren zur Entfernung von Ammoniumverbindungen bekannt, wobei die Ammoniumverbindung mit einem synthetischen Aluminiumsilicat oder einem Tonmineral, wie Bentonit, der zuvor amorph gemacht wurde, in Berührung gebracht wird. Das Absorptionsmittel wird nach Gebrauch durch Behandlung mit einer alkalischen Lösung regeneriert, wobei die gebundenen Ammoniumionen durch Alkaliionen ausgetauscht werden.

Aus der US-A-5 037 412 ist ein Sorptionskissen für Körperflüssigkeiten bekannt, das in einer Umhüllung ein Sorptionsmittel, z.B. ein Gemisch aus Citronensäure, Natriumbicarbonat und Zeolith, enthält. Bentonit ist nicht erwähnt.

Aus der US-A-4 641 605 ist eine Tierstreu bekannt, die die Freisetzung von Ammoniak verzögert. Die Tierstreu enthält zu diesem Zweck ein Sorptionsmittel, wie Bentonit, das mit Oxidationsmitteln, wie Alkalipersulfaten und Ammoniumpersulfat, vermischt ist. Diese dienen nicht als Aktivierungsmittel.

Aus der US-A-4 913 835 ist ein Mittel zur Neutralisation und Verfestigung von verschütteten, gefährlichen alkalischen Flüssigkeiten bekannt, das ein Gemisch aus schwachen organischen Säuren, einem Tonmineral und einem wasserlöslichen, schwach sauren Salz darstellt. Eine Aktivierung des Tonminerals wird nicht durchgeführt.

Aus der US-A-4 949 672 und der US-A-5 176 108 sind Streumaterialien auf der Basis von Bentonit bekannt, die mit einem flüssigen Träger getränkt sind, der eine bakterizide Borverbindung sowie ein Alkalihydroxid enthält. Eine Aktivierung des Bentonits durch Verkneten ist nicht beschrieben.

Weiterhin sind z.B. aus den US-A-5 000115 und 4 657881 bzw. aus der EP-A-0 378 421 Streumaterialien auf Bentonitbasis bekannt, die gegenüber den organischen Streumaterialien gewisse Vorteile haben. Durch die Fähigkeit zur Quellung mit wäßrigen Flüssigkeiten sind Bentonite in der Lage, Klumpen zu bilden. Wegen dieser Klumpenbildung kann der mit Flüssigkeit benetzte Anteil des Streumaterials separat entfernt werden, wodurch Streumaterial eingespart werden kann. Mit der besonderen Struktur der Bentonite ist auch die Fähigkeit zur Adsorption von störenden Gerüchen verbunden, die bei den tierischen Ausscheidungen und Exkrementen entstehen. Bentonite lassen sich weiterhin umweltverträglich entsorgen.

Nach dem vorstehend genannten Stand der Technik werden u.a. Natriumbentonite bzw. Mischungen aus Natriumbentoniten und Calciumbentoniten als Sorptionsmittel, z.B. als Streumaterial für Heimtiere verwendet. Diese Sorptionsmittel können in granulierter Form eingesetzt werden.

Natriumbentonite sind jedoch nur regional verfügbar und werden aus diesem Grund meist nur für Spezialanwendungen, z.B. als Bindemittel für Gießereiformsand und als Verdickungs- und Thixotropiermittel für wäßrige Medien gebraucht. Hochwertige Calciumbentonite können für diese Zwecke aktiviert werden, wobei aber große Mengen an alkalischen Aktivierungsmitteln (z.B. mindestens 3,5 % Natriumcarbonat, bezogen auf den trockenen Ton) benötigt werden, um die erforderliche Bindefähigkeit zu erreichen. Andererseits gibt es in großen Mengen Calciumbentonite, die wegen des niedrigen Gehalts an dem Hauptmineral Montmorillonit auch nach einer alkalischen Aktivierung für diese Spezialanwendungen nicht geeignet sind.

Der Erfindung liegt die Aufgabe zugrunde, diese bisher nicht verwendbaren und zum Teil als Abraum beseitigten Bentonite mit niedrigem Montmorillonitgehalt einer wirtschaftlichen und ökologisch sinnvollen Nutzung zuzuführen.

Die Erfindung betrifft somit ein Verfahren zur Herstellung von Sorptionsmitteln auf Bentonitbasis, zur Aufnahme von Flüssigkeiten, das dadurch gekennzeichnet ist, daß man einen schwach quellenden Bentonit mit einer Wasseraufnahmefähigkeit von weniger als 110 %, vorzugsweise weniger als 115 % (bezogen auf den getrockneten Bentonit mit einem Restwassergehalt von 6 Gew.-%), einem Montmorillonitgehalt von etwa 40 bis 65 Gew.-%, vorzugsweise von 50 bis 60 Gew.-% und einem Wassergehalt von 20 bis 40 Gew.-%, vorzugsweise von 25 bis 35 Gew.-%, mit einer basisch reagierenden Alkalimetallverbindung durch innige Verknetung homogenisiert und unter Ionenaustausch in einen quellfähigen Bentonit überführt, wobei der pH-Wert der Mischung nicht mehr als 10,5, vorzugsweise nicht mehr als 10,0 beträgt, die Mischung schonend trocknet und die getrocknete Mischung zerkleinert.

Der Wassergehalt, die Wasseraufnahmefähigkeit und der pH-Wert werden nach den nachstehend angegebenen Methoden bestimmt.

Die als Ausgangsmaterial verwendeten schwachquellenden Bentonite konnten bisher noch keiner sinnvollen Nutzung zugeführt werden und mußten verworfen werden. Das niedrige Quellvermögen ist in erster Linie dadurch bedingt, daß diese Bentonite nicht-quellende Bestandteile, wie Quarz, Kaolin, Glimmer, Feldspäte, Calcit und Dolomit enthalten. Ferner ist das niedrige Quellvermögen auch dadurch bedingt, daß der Montmorillonit hauptsächlich in Form von Calcium-Montmorillonit vorliegt. Bei der Verknetung des Bentonits mit der basisch reagierenden Alkalimetallverbindung findet in erster Linie ein Austausch der Calciumionen in den Zwischenschichten des Montmorillonits durch Alkaliionen, insbesondere Natriumionen, statt. Die Energieaufnahme bei der Verknetung beträgt im allgemeinen 2 bis 10, vorzugsweise 3 bis 6 kWh/to Knetgemisch.

Durch den Ionenaustausch wird die Quellfähigkeit und damit die Wasseraufnahmefähigkeit des Bentonits stark erhöht, so daß er nach dem Trocknen in der Lage ist, größere Mengen an Flüssigkeiten aufzunehmen. Die Trocknung des alkalisierten Bentonits erfolgt schonend ohne schädigenden Einfluß auf die Wasseraufnahmefähigkeit des Bentonits. Diese Bedingungen kann man herstellen, wenn das Material mit nicht mehr als 150°C, vorzugsweise mit nicht mehr als 120°C belastet wird und der Restwassergehalt nicht unter 6 Gew.-%, vorzugsweise nicht unter 4 Gew.-% fällt.

Die getrocknete Mischung liegt im allgemeinen in Form von größeren Stücken vor, die in üblicher Weise zerkleinert werden. Die beim Zerkleinern entstehenden Feinanteile werden in üblicher Weise abgesiebt und in die Knetvorrichtung zurückgeleitet.

Im allgemeinen geht man von einem Erdalkalibentonit, insbesondere von Calciumbentonit, aus, welcher im trockenen Zustand eine Wasseraufnahmefähigkeit von mindestens etwa 60 % (bezogen auf getrockneten Rohton mit einem Restfeuchtigkeitsgehalt von 6 Gew.-%) aufweist.

Als basisch reagierende Alkalimetallverbindungen verwendet man vorzugsweise die wasserlöslichen Natriumsalze von schwachen bis mittelstarken Säuren, wie Kohlensäure, Kieselsäure, Oxalsäure, Citronensäure, Phosphorsäure oder Essigsäure.

Vorzugsweise setzt man die basisch reagierende Alkalimetallverbindung (vorzugsweise die entsprechende Natriumverbindung) in einer Menge von 0,1 bis 1,5 %, vorzugsweise von 0,25 bis 1,5 %, bezogen auf den getrockneten Rohbentonit (atro = absolut trocken) ein.

Überraschenderweise wurde gefunden, daß bereits diese geringen Zusätze in der Lage sind, das Wasseraufnahmevermögen des so behandelten Bentonits erheblich zu erhöhen. Größere Zusätze, wie sie z.B. bei der Aktivierung von Gießereibentonit angewendet werden, sind ungünstig, da sie nur den pH-Wert des Materials erhöhen, was für seine Verwendung als Streumaterial für Heimtiere ungünstig ist, weil hierbei uangenehme Gerüche entstehen können.

Um einen möglichst intensiven Ionenaustausch zu erzielen, geht man vorzugsweise so vor, daß man die basisch reagierende Alkalimetallverbindung in fester Form oder in Form von wäßrigen Lösungen in den Bentonit einknetet. Diese Behandlung kann bei Raumtemperatur durchgeführt werden, kann aber durch Temperaturerhöhung beschleunigt werden.

Die Wasseraufnahmefähigkeit des unbehandelten Materials, die gewöhnlich unter 115 % liegt, kann durch die erfindungsgemäße Behandlung mehr als verdoppelt werden. Durch die Auswahl geeigneter Alkalimetallverbindungen und Variation der in diesen enthaltenen Anionen kann neben der Vervielfachung der Wasseraufnahmefähigkeit durch Aktivierung zusätzlich eine Einstellung der basischen Eigenschaften bzw. des pH-Wertes des behandelten Materials erfolgen. Weiterhin wird durch die Aktivierung eine Verbesserung der Klumpenbildung und eine Erhöhung der Klumpenkonsistenz des mit Flüssigkeit getränkten Materials durch Optimierung des Quellverhaltens erreicht.

Eine weitere Optimierung der Flüssigkeitsaufnahme kann dadurch erreicht werden, daß man das getrocknete Produkt auf eine Korngröße von etwa 0,1 bis 10 mm, vorzugsweise von 1 bis 5 mm aufarbeitet.

Gegenstand der Erfindung ist ferner ein Sorptionsmittel, das nach dem vorstehend beschriebenen Verfahren erhältlich ist und das durch einen Wassergehalt von 3 bis 12, vorzugsweise von 4 bis 10 Gew.-%, eine Wasseraufnahmefähigkeit von mehr als 120 %, vorzugsweise von mehr als 150 % (bezogen auf das getrocknete Material mit einem Restwassergehalt von 6 %), und einen pH-Wert (gemessen in einer 8 %igen wäßrigen Suspension) von 7,5 bis 10,5, vorzugsweise von 8,0 bis 10, gekennzeichnet ist.

Das erfindungsgemäße Sorptionsmittel kann ferner mit an sich bekannten Sorptionsmitteln, z.B. den vorstehend angegebenen organischen Sorptionsmitteln, verschnitten werden. Weiterhin kann das Sorptionsmittel Weißpigmente, Desinfektionsmittel und/oder Tierakzeptanzien enthalten.

Gegenstand der Erfindung ist ferner die Verwendung des vorstehend angegebenen Sorptionsmittels als Aufsaugmittel für Flüssigkeiten, wie Körperflüssigkeiten, ÖL oder flüssige Chemikalien, sowie als Streumaterial für Heimtiere, insbesondere als Katzenstreu.

Der Wassergehalt des erfindungsgemäßen Sorptionsmittels wird wie folgt bestimmt:

10 g Sorptionsmittel werden in einer flachen Schale auf 0,01 g genau eingewogen und im Trockenschrank bei 110°C bis zur Gewichtskonstanz getrocknet (mindestens 2 Stunden). Anschließend läßt man die Probe im Exsikkator auf Raumtemperatur abkühlen und wiegt aus:

Auswertung:

$$\frac{\text{Einwaage - Auswaage}}{\text{Einwaage}} \times 100 = \text{Wassergehalt (\%)}$$

Die Wasseraufnahmefähigkeit des erfindungsgemäßen Sorptionsmittels wird nach der Methode der Firma Westinghouse (Nr. 17-A) ermittelt. (Vergl. Industrial Minerals, August 1992, S. 57). Hierbei wird das bis auf einen Restwassergehalt von 6 Gew.-% getrocknete Sorptionsmittel in einen kegeligen Behälter aus feinem Drahtgewebe (lichte Maschenweite 0,25 mm = 60 mesh, Durchmesser 7 cm, Höhe 7,6 cm) eingewogen (Einwaage E = 20 g). Anschließend wird das Gesamtgewicht (Drahtgewebe + Einwaage E = $E_1$ in g) ermittelt. Das gefüllte Gewebe wird 20 Minuten in eine mit Wasser gefüllten Glasschale derart eingehängt, daß das Sorptionsmittel vollständig untertaucht. Nach 20 Minuten nimmt man das Drahtgewebe aus dem Wasser und läßt es 20 Minuten abtropfen. Unmittelbar danach wird das Gewicht des Behälters mit Inhalt ermittelt ($E_2$ in g). Die Auswertung wird wie folgt durchgeführt:

$$\text{Wasseraufnahme in Prozent} = (E_2 - E_1)/E \times 100$$

Der pH-Wert des erfindungsgemäßen Sorptionsmittels wird wie folgt bestimmt:

In ein Becherglas mit 1000 ml destilliertem Wasser werden 80 g Sorptionsmittel (Wassergehalt 6 Gew.-%) gegeben. Nach einer Standzeit von einer Stunde wird 10 Minuten mit einem Wirbler gerührt. Die Suspension wird 24 Stunden stehengelassen. Vor der Prüfung wird erneut etwa 30 Sekunden gerührt.

Das pH-Wert-Meßgerät wird mit zwei Pufferlösungen mit den pH-Werten 7 und 9 geeicht. Dazu wird die Elektrode zunächst etwa 30 Sekunden in die Lösung mit dem pH-Wert 7 gegeben. Die Elektrode wird herausgenommen und mit destilliertem Wasser abgespült. Dann wird sie in die Lösung mit dem pH-Wert 9 gegeben; dieser wird wie oben eingestellt. Die Elektrode wird wiederum mit destilliertem Wasser abgespült und in die Bentonitsuspension gegeben.

Die Erfindung ist durch die nachstehenden Beispiele in nicht einschränkender Weise erläutert.

Beispiel 1 (Vergleich)

Etwa 2 kg grubenfeuchter Ca-Rohbentonit (etwa 30 Gew.-% Wasser) mit einem Montmorillonitgehalt von 60 %, bezogen auf die Trockenmasse, und einer Wasseraufnahmefähikgeit von 110 % (bezogen auf ein getrocknetes Material mit einem Restwassergehalt von 6 Gew.-%) werden in einem Knetwerk mit Scherwirkung (Werner-Pfleiderer-Mischer) 5 Minuten intensiv geknetet. Die Energieaufnahme beträgt 2kWh/to. Die erhaltenen Agglomerate werden schonend bei 75°C 4 Stunden getrocknet und bei einem Wassergehalt von 6 Gew.-% auf eine Korngröße von 1 - 5 mm zerkleinert. Von den Granulaten werden die Wasseraufnahmefähigkeit sowie der pH-Wert in Suspension nach den vorstehend beschriebenen Methoden gemessen.

Die erhaltenen Werte sind in der nachstehenden Tabelle angegeben.

Beispiel 2

Die Arbeitsweise von Beispiel 1 wird unter Zusatz von Natriumcarbonat in fester Form wiederholt. Die zugesetzten Mengen entsprechen 0,29 %, 0,58 % und 1,75 % $Na_2O$ (bezogen auf Bentonit-Trockensubstanz).

Beispiel 3

Die Arbeitsweise von Beispiel 1 wird unter Zusatz von Natriumoxalat in fester Form wiederholt. Die zugesetzten Mengen entsprechen einem $Na_2O$-Gehalt von 0,23 %, 046 % und 1,39 % (bezogen auf Bentonit-Trockensubstanz).

Beispiel 4

Die Arbeitsweise von Beispiel 1 wird unter Zusatz von Natriumcitrat in fester Form wiederholt. Die zugesetzten Mengen entsprechen 0,13%, 0,26 % und 0,78 % $Na_2O$ (bezogen auf Bentonit-Trockensubstanz).

Beispiel 5

Die Arbeitsweise von Beispiel 1 wird unter Zusatz Natriumacetat in fester Form wiederholt. Die zugesetzten Mengen entsprechen 0,19 %, 0,38 % und 1,14 % $Na_2O$ (bezogen auf Bentonit-Trockensubstanz).

Beispiel 6

Die Arbeitsweise von Beispiel 1 wird unter Zusatz einer wäßrigen Natriumcarbonatlösung wiederholt. Die zugesetzten Mengen entsprechen 0,29 %, 0,58 %, 1,16 % und 1,75 % $Na_2O$ (bezogen auf Bentonit-Trockensubstanz).

Beispiel 7

Die Arbeitsweise von Beispiel 1 wird unter Zusatz einer wäßrigen Natriumoxalatlösung wiederholt. Die zugesetzten Mengen entsprechen 0,28 %, 0,60 %, 1,16 % und 1,76 % $Na_2O$ (bezogen auf Bentonit-Trockensubstanz).

Beispiel 8

Die Arbeitsweise von Beispiel 1 wird unter Zusatz einer wäßrigen Natriumcitratlösung wiederholt. Die zugesetzten Mengen entsprechen 0,29 %, 0,60 % und 1,17 % $Na_2O$ (bezogen auf Bentonit-Trockensubstanz).

Beispiel 9

Die Arbeitsweise von Beispiel 1 wird unter Zusatz einer Wasserglaslösung wiederholt. Die zugesetzten Mengen entsprechen einem $Na_2O$-Gehalt von 0,5 %, 1,0 % und 1,5 %.

Tabelle

| Bsp. | Bentonit-Aktivierung | Zugabe berechnet als $Na_2O$(%) | Wasseraufnahmefähig-keit (%) | pH-Wert |
|------|---------------------|------------------------|------------------------------|---------|
| 1 | ohne Zusatz | | 110 | 8,6 |
| 2 | Na-Carbonat (in fester Form) | 0,29 | 150 | 9,3 |
| | | 0,58 | 210 | 9,7 |
| | | 1,75 | 250 | 10,7 |
| 3 | Na-Oxalat (in fester Form) | 0,23 | 130 | 8,6 |
| | | 0,46 | 190 | 8,7 |
| | | 1,39 | 250 | 8,9 |
| 4 | Na-Citrat (in fester Form) | 0,13 | 110 | 9,0 |
| | | 0,26 | 110 | 9,0 |
| | | 0,78 | 150 | 9,1 |
| 5 | Na-Acetat (in fester Form) | 0,19 | 140 | 9,2 |
| | | 0,38 | 130 | 9,1 |
| | | 1,14 | 140 | 9,0 |
| 6 | Na-Carbonat (in gelöster Form) | 0,29 | 170 | 9,5 |
| | | 0,58 | 190 | 9,7 |
| | | 1,16 | 280 | 10,1 |
| | | 1,75 | 320 | 10,4 |
| 7 | Na-Oxalat (in gelöster Form) | 0,28 | 160 | 8,8 |
| | | 0,60 | 180 | 9,0 |
| | | 1,16 | 300 | 9,2 |
| | | 1,76 | 280 | 9,6 |
| 8 | Na-Citrat (in gelöster Form) | 0,29 | 130 | 8,8 |
| | | 0,60 | 160 | 9,0 |
| | | 1,17 | 180 | 9,1 |
| 9 | Na-Silicat (in gelöster Form) | 0,5 | 168 | 9,2 |
| | | 1,0 | 231 | 9,7 |
| | | 1,5 | 241 | 10,0 |

**Patentansprüche**

1. Verfahren zur Herstellung von Sorptionsmitteln auf Bentonitbasis, zur Aufnahme von Flüssigkeiten, dadurch gekennzeichnet, daß man einen schwach quellenden Bentonit mit einer Wasseraufnahmefähigkeit von weniger als 100 %, (bezogen auf den getrockneten Bentonit mit einem Restwassergehalt von 6 Gew.-%), einem Montmorillonitgehalt von etwa 40 bis 65 Gew.-% und einem Wassergehalt von etwa 20 bis 40 Gew.-% mit einer basisch reagierenden Alkalimetallverbindung durch innige Verknetung homogenisiert und unter Ionenaustausch in einen quellfähigen Bentonit überführt, wobei der pH-Wert der Mischung nicht mehr als 10,5 beträgt, die Mischung schonend trocknet und die getrocknete Mischung zerkleinert.

2. Verfahren nach Anspruch 1, dadurch gekennzeichnet, daß man einen Bentonit mit einer Wasseraufnahmefähigkeit von weniger als 115 % (bezogen auf den getrockneten Bentonit mit einem Restwassergehalt von 6 Gew.-%), einem Montmorillonitgehalt von etwa 50 bis 60 Gew.-% und einem Wassergehalt von etwa 25 bis 35 Gew.-% verwendet und daß der pH-Wert der Mischung nicht mehr als 10,0 beträgt.

3. Verfahren nach Anspruch 1 oder 2, dadurch gekennzeichnet, daß man die Verknetung bei einer Energieaufnahme von 2 bis 10, vorzugsweise von 3 bis 6 kWh/to Knetgemisch durchführt.

4. Verfahren nach einem der Ansprüche 1 bis 3, dadurch gekennzeichnet, daß man das Material beim Trocknen mit nicht mehr als 150°C, vorzugsweise mit nicht mehr als 120°C belastet und den Restwassergehalt auf nicht weniger als 6 Gew.-%, vorzugsweise nicht weniger als 4 Gew.-% einstellt.

5. Verfahren nach einem der Ansprüche 1 bis 4, dadurch gekennzeichnet, daß man von einem Erdalkalibentonit, vorzugsweise von Calciumbentonit, ausgeht, welcher eine Wasseraufnahmefähigkeit von mindestens etwa 60 % (bezogen auf getrockneten Rohton mit einem Restfeuchtigkeitsgehalt von 6 Gew.-%) aufweist.

6. Verfahren nach einem der Ansprüche 1 bis 5, dadurch gekennzeichnet, daß man als basisch reagierende Alkalimetallverbindungen die wasserlöslichen Natriumsalze von schwachen bis mittelstarken Säuren, wie Kohlensäure, Kieselsäure, Oxalsäure, Citronensäure oder Essigsäure verwendet.

7. Verfahren nach einem der Ansprüche 1 bis 6, dadurch gekennzeichnet, daß man die basisch reagierende Alkalimetallverbindung in einer Menge von 0,1 bis 1,5 %, vorzugsweise von 0,25 bis 1,5 %, bezogen auf den getrockneten Rohbentonit (atro = absolut trocken) einsetzt.

8. Verfahren nach einem der Ansprüche 1 bis 7, dadurch gekennzeichnet, daß man die basisch reagierende Alkalimetallverbindung in fester Form oder in Form von wäßrigen Lösungen in den Bentonit einknetet.

9. Verfahren nach einem der Ansprüche 1 bis 8, dadurch gekennzeichnet, daß man das getrocknete Produkt auf eine Korngröße von etwa 0,1 bis 10 mm, vorzugsweise von 1 bis 5 mm aufarbeitet.

10. Sorptionsmittel, das nach dem Verfahren nach einem der Ansprüche 1 bis 9 erhältlich ist und das durch einen Wassergehalt von 3 bis 12, vorzugsweise von 4 bis 10 Gew.-%, eine Wasseraufnahmefähigkeit von mehr als 120 %, vorzugsweise von mehr als 150 Gew.-%, (bezogen auf das getrocknete Material mit einem Restwassergehalt von 6 Gew.-%) und einem pH-Wert (gemessen in einer 8 %igen wäßrigen Suspension) von 7,5 bis 10,5, vorzugsweise von 8,0 bis 10, gekennzeichnet ist.

11. Sorptionsmittel nach Anspruch 10, dadurch gekennzeichnet, daß es mit an sich bekannten Sorptionsmitteln, z.B. mit organischen Sorptionsmitteln, verschnitten ist.

12. Sorptionsmittel nach Anspruch 10 oder 11, dadurch gekennzeichnet, daß es Weißpigmente, Desinfektionsmittel und/oder Tierakzeptanzien enthält.

13. Verwendung des Sorptionsmittels nach einem der Ansprüche 10 bis 12 als Aufsaugmittel für Flüssigkeiten, wie Körperflüssigkeiten, Öl oder flüssige Chemikalien, sowie als Streumaterial für Heimtiere, insbesondere als Katzenstreu.

## Claims

1. A method of preparing sorbents based on bentonite for the absorption of liquids, characterised in that a weakly swelling bentonite with a water-absorption capacity of less than 100 % (in relation to the dried bentonite with a residual water content of 6 %), a montmorillonite content of approximately 40 to 65 % by weight and a water content of approximately 20 to 40 % by weight is homogenised with a basic-reacting alkaline metal compound by intimate kneading and is converted by ion exchange into a bentonite capable of swelling, wherein the pH value of the mixture is not more than 10.5, the mixture is dried gently and the dried mixture is comminuted.

2. A method according to Claim 1, characterised in that a bentonite with a water-absorption capacity of less than 115 % (in relation to the dried bentonite with a residual water content of 6 %), a montmorillonite content of approximately 50 to 60 % by weight and a water content of approximately 25 to 35 % by weight is used, and in that the pH value of the mixture is not more than 10.0.

3. A method according to Claim 1 or 2, characterised in that the kneading is carried out with an energy consumption of 2 to 10, preferably 3 to 6 kWh/ton of kneading mixture.

4. A method according to any one of Claims 1 to 3, characterised in that during drying the material is exposed to a temperature of not more than 150°C, preferably not more than 120°C, and the residual water content is adjusted to not less than 6 % by weight, preferably not more than 4 % by weight.

5. A method according to any one of Claims 1 to 4, characterised in that the starting material is an alkaline earth bentonite, preferably calcium bentonite, which has a water-absorption capacity of at least approximately 60 % (in relation to crude dried clay with a residual moisture content of 6 % by weight.

6. A method according to any one of Claims 1 to 5, characterised in that the basic-reacting alkaline metal compounds used are the water-soluble sodium salts of weak to medium strong acids, such as carbonic acid, silicic acid, oxalic acid, citric acid or acetic acid.

7. A method according to any one of Claims 1 to 6, characterised in that the basic-reacting alkaline metal compound is used in a quantity of 0.1 to 1.5 %, preferably 0.25 to 1.5 %, in relation to the crude dried bentonite (atro = absolutely dry).

8. A method according to any one of Claims 1 to 7, characterised in that the basic-reacting alkaline metal compound is kneaded into the bentonite in solid form or the form of aqueous solutions.

9. A method according to any one of Claims 1 to 8, characterised in that the dried product is processed to a grain size of approximately 0.1 to 10 mm, preferably 1 to 5 mm.

10. A sorbent which can be obtained in accordance with the method of any one of Claims 1 to 9 and which is characterised by a water content of 3 to 12, preferably 4 to 10 % by weight, a water-absorption capacity of more than 120 %, preferably of more than 150 % by weight (in relation to the dried material with a residual water content of 6 % by weight) and a pH value (measured in an 8 % aqueous suspension) of 7.5 to 10.5, preferably of 8.0 to 10.

11. A sorbent according to Claim 10, characterised in that it is blended with per se known sorbents, for example with organic sorbents.

12. A sorbent according to Claim 10 or 11, characterised in that it contains white pigments, disinfectants and/or animal acceptant agents.

13. Use of the sorbent according to any one of Claims 10 to 12 as an absorbent for fluids, such as body fluids, oil or liquid chemicals, and also as litter for pets, in particular as cat litter.

**Revendications**

1. Procédé de préparation de sorbants à base de bentonite, pour absorber des liquides, caractérisé en ce qu'on homogénéise une bentonite à faible pouvoir gonflant, ayant un pouvoir d'absorption de l'eau inférieur à 100 %, (par rapport à la bentonite séchée ayant une teneur résiduelle en eau de 6 % en poids), une teneur en montmorillonite d'environ 40 à 65 % en poids, et une teneur en eau de 20 à 40 % en poids, par malaxage intime, avec un composé à réaction basique d'un métal alcalin, et, par échange d'ions, on le transforme en une bentonite gonflable, le pH du mélange n'étant pas supérieur à 10,5, on sèche le mélange dans des conditions ménagées et on broie le mélange séché.

2. Procédé selon la revendication 1, caractérisé en ce qu'on utilise une bentonite ayant un pouvoir d'absorption de l'eau inférieur à 115 % (par rapport à la bentonite séchée ayant une teneur résiduelle en eau de 6 % en poids), une teneur en montmorillonite d'environ 50 à 60 % en poids et une teneur en eau d'environ 25 à 35 % en poids, et que le pH du mélange n'est pas supérieur à 10,0.

3. Procédé selon la revendication 1 ou 2, caractérisé en ce qu'on met en oeuvre le malaxage en présence d'une énergie absorbée de 2 à 10 et de préférence de 3 à 6 kwh/t de mélange à malaxer.

4. Procédé selon l'une des revendications 1 à 3, caractérisé en ce qu'on soumet le matériau, lors du séchage, à une température non supérieure à 150°C, de préférence non supérieure à 120°C, et on ajuste la teneur résiduelle en eau à une valeur non inférieure à 6 % en poids et de préférence non inférieure à 4 % en poids.

5. Procédé selon l'une des revendications 1 à 4, caractérisé en ce qu'on part d'une bentonite alcalino-terreuse, de préférence de bentonite calcique qui présente un pouvoir d'absorption de l'eau d'au moins environ 60 % (par rapport à l'argile brute séchée ayant une teneur résiduelle en humidité de 6 % en poids).

6. Procédé selon l'une des revendications 1 à 5, caractérisé en ce qu'on utilise en tant que composés à réaction basique de métaux alcalins les sels de sodium solubles dans l'eau d'acides faibles à moyennement forts tels que l'acide carbonique, l'acide silicique, l'acide oxalique, l'acide citrique ou l'acide acétique.

7. Procédé selon l'une des revendications 1 à 6, caractérisé en ce qu'on utilise le composé à réaction basique d'un métal alcalin en une quantité de 0,1 à 1,5 % et de préférence de 0,25 à 1,5 % par rapport a la bentonite brute séchée (s.a. = sec absolu).

8. Procédé selon l'une des revendications 1 à 7, caractérisé en ce qu'on malaxe dans la bentonite le composé à réaction basique d'un métal alcalin sous forme solide ou sous forme de solutions aqueuses.

9. Procédé selon l'une des revendications 1 à 8, caractérisé en ce qu'on traite le produit séché jusqu'à une granulométrie d'environ 0,1 à 10 mm, et de préférence de 1 à 5 mm.

10. Sorbant pouvant être préparé par le procédé selon l'une des revendications 1 à 9, et qui est caractérisé par une teneur en eau de 3 à 12 et de préférence de 4 à 10 % en poids, par un pouvoir d'absorption de l'eau supérieur à 120 % et de préférence supérieur à 150 % en poids (par rapport au matériau séché ayant une teneur résiduelle en eau de 6 % en poids) et par un pH (mesuré dans une suspension aqueuse à 8 %) de 7,5 à 10,5 et de préférence de 8,0 à 10.

11. Sorbant selon la revendication 10, caractérisé en ce qu'il est dilué avec des sorbants connus en soi, par exemple avec des sorbants organiques.

12. Sorbant selon la revendication 10 ou 11, caractérisé en ce qu'il contient des pigments blancs, des désinfectants et/ou des attractifs pour animaux.

13. Utilisation du sorbant selon l'une des revendications 10 à 12 en tant que matériau absorbant pour liquides, tels que les fluides corporels, l'huile ou les produits chimiques liquides, ou comme matériau de litières pour animaux domestiques, en particulier comme litière pour chats.